# FASCICULE DE BREVET EUROPEEN

(11) **EP 0 752 401 B1**
(45) Date de publication et mention de la délivrance du brevet: **26.04.2000**
(21) Numéro de dépôt: 96401390.8
(22) Date de dépôt: 24.06.1996
(51) Int. Cl.: C07C 2/62

(54) **Procédé d'alkylation aliphatique en émulsion inverse avec prémélange catalyseur-olèfine**
Verfahren zur aliphatischen Alkylierung in inverser Emulsion mit Vormischung von Katalysator und Olefinen
Process for aliphatic alkylation in inverse emulsion with premixing of the catalyst and the olefin

(30) Priorité: 06.07.1995 FR 9508294
(43) Date de publication de la demande: 08.01.1997
(73) Titulaire: INSTITUT FRANCAIS DU PETROLE, 92502 Rueil-Malmaison (FR)
(72) Inventeur: Alagy, Jacques, 69260 Charbonnieres (FR); Joly, Jean-François, 69006 Lyon (FR); Benazzi, Eric, 78360 Montesson (FR); Viltard, Jean-Charles, 26000 Valence (FR); Forestiere, Alain, 69390 Vernaison (FR); Borges, Luis, 22450-140 Rio de Janeiro (BR)

(56) Documents cités:
- WO-A-95/04019
- FR-A- 1 176 974

## Description

La présente invention concerne un procédé d'alkylation catalytique d'au moins une isoparaffine choisie dans le groupe formé par l'isobutane et l'isopentane par au moins une oléfine comprenant de 2 à 6, de préférence de 3 à 6 atomes de carbone par molécule, en présence d'au moins un catalyseur acide liquide.

L'alkylation d'isoparaffine (isobutane et/ou isopentane) par au moins une oléfine contenant de 2 à 6, de préférence de 3 à 6 atomes de carbone par molécule permet d'obtenir des hydrocarbures paraffiniques fortement ramifiés (par exemple appartenant au groupe constitué par les diméthylbutanes, les triméthylpentanes, les triméthylhexanes et les triméthylheptanes), constituants essentiels des carburants à haut indice d'octane. Cette réaction d'alkylation dite aliphatique nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Un grand nombre de catalyseurs acides, liquides ou solides, sont connus pour réaliser l'alkylation aliphatique d'isoparaffine(s) telles que l'isobutane ou l'isopentane, par au moins une oléfine telle que le propylène, les butènes-1 et -2, l'isobutène. Les catalyseurs les plus couramment utilisés dans la pratique industrielle sont les catalyseurs liquides que sont l'acide sulfurique concentré et l'acide fluorhydrique seul ou en mélange avec des acides de Lewis tels que le trifluorure de bore.

La plupart des procédés conventionnels sont en particulier caractérisés en ce que la phase acide constitue la phase continue de l'émulsion acide-hydrocarbure formée au sein du réacteur. Le rapport des volumes d'acide et d'hydrocarbures (mélange d'isoparaffines et d'oléfines) est donc supérieur à 1. De plus, l'émulsion est généralement réalisée au sein même du réacteur d'alkylation, c'est-à-dire en présence de la charge à convertir qui contient l'oléfine (voir par exemple dans le cas de la mise en oeuvre de la technologie Stratco employant l'acide sulfurique liquide : L.F. Albright, Chem. Eng., August 15, 1966, p. 143 et L.F. Albright, Oil & Gas Journal, November 12, 1990).

Dans le cas où l'acide sulfurique est le catalyseur, le procédé conventionnel d'alkylation d'isoparaffines avec l'acide sulfurique comme catalyseur présente de nombreux inconvénients parmi lesquels on peut citer :
- l'impossibilité d'atteindre des températures inférieures à 0°C dans le réacteur en présence d'acide sulfurique de titre supérieur à 97% poids. En effet la viscosité de l'acide sulfurique devient trop élevée à de telles températures et, l'acide étant la phase continue, le milieu devient inagitable.
- l'oléfine de la charge et l'acide recyclé du décanteur sont injectés directement dans le réacteur au voisinage immédiat du mobile d'agitation. Ce dernier doit donc réaliser deux opérations : créer l'émulsion acide-hydrocarbure et assurer la dilution de l'oléfine dans la phase hydrocarbonée de façon à limiter les surconcentrations locales en la dite oléfine, ce qui n'est pas réalisé de façon suffisamment efficace.
- le temps de séjour de la phase hydrocarbure dans le décanteur est important, le plus souvent voisin de 1 heure. La température dans ledit décanteur étant le plus souvent supérieure à environ 15°C, il se produit alors des réactions de dégradation des isoparaffines ayant plus de 5 atomes de carbone par molécule. Parmi ces réactions de dégradation, on peut citer les réactions d'oxydation des paraffines par l'acide sulfurique qui produisent de l'eau et du SO₂. Ces réactions contribuent à la désactivation du catalyseur. On peut également citer les réactions de décomposition non contrôlées des sulfates d'alkyle et en particulier des sulfates de butyle. Ces réactions de décomposition sont à l'origine de la formation d'oligomères insaturés qui désactivent le catalyseur.

La demande de brevet PCT WO 95/04.019 décrit, elle, un procédé d'alkylation d'une oléfine par une isoparaffine en présence d'acide sulfurique liquide, le procédé comprenant une zone de préparation d'une émulsion de l'acide dans l'isoparaffine suivie d'une zone de réaction alimentée par ladite émulsion dans laquelle on injecte l'oléfine, le rapport volumique acide sulfurique: hydrocarbures présents dans la zone de réaction étant compris entre 0,3:1 et 0,5 et 1. Ainsi la phase continue de l'émulsion préparée dans ledit procédé est la phase hydrocarbonée. Ladite demande de brevet décrit aussi les moyens de réalisation de l'émulsion et de l'apport d'oléfine en zone réactionnelle, ainsi que l'appareillage de mise en oeuvre du procédé.

Dans la demande de brevet PCT WO 95/04.019, l'oléfine est injectée directement dans la zone réactionnelle. Ladite zone doit donc assurer la dilution de l'oléfine dans la phase hydrocarbonée de façon à limiter les surconcentrations locales en la dite oléfine.

Or la réaction d'alkylation d'isobutane et/ou isopentane comprend une première étape très rapide d'adorption de l'oléfine sur le catalyseur acide, permettant la formation d'une sorte de "complexe" acide-oléfine, suivie d'une étape de réaction dudit "complexe" avec l'isobutane et/ou l'isopentane. Et ladite première étape, si elle est menée dans les conditions réactionnelles de pression et de température, peut favoriser la formation de polymères, produits secondaires non désirés, au détriment de la formation dudit "complexe". Ceci est très pénalisant quant au rendement du procédé. Il est donc intéressant de procéder à un mélange de l'oléfine et du catalyseur dans une zone qui précède la zone réactionnelle, dans des conditions de température et de pression qui favorisent principalement la formation du "complexe".

Le procédé selon l'invention est donc un procédé d'alkylation d'au moins une isoparaffine choisie dans le groupe formé par l'isobutane et l'isopentane, de préférence l'isobutane, par au moins une oléfine comprenant de 2 à 6, de préférence de 3 à 6 atomes de carbone par molécule, en présence d'un catalyseur acide liquide, ledit procédé comprenant le mélange dans une zone de premier mélange de la charge comprenant l'oléfine à convertir et d'un effluent comprenant en majeure partie de l'isoparaffine, permettant l'obtention d'une charge diluée comprenant l'oléfine, ainsi que la formation dans une zone d'émulsion d'une émulsion dudit catalyseur dans un effluent hydrocarboné comprenant en majeure partie de l'isoparaffine, ledit effluent constituant la phase continue de l'émulsion ainsi formée, puis le mélange dans une zone de second mélange de la majeure partie de l'émulsion de l'acide dans l'effluent hydrocarboné et de la majeure partie de la charge diluée comprenant l'oléfine, suivie de la mise en oeuvre de la majeure partie de la réaction dans une zone de réaction qui est alimentée en la majeure partie dudit mélange, la température de la zone de second mélange étant inférieure à la température de la zone de réaction.

Les opérations de premier mélange et de formation d'émulsion peuvent avoir lieu simultanément ou non, l'ordre desdites opérations important peu lorsqu'elles sont réalisées successivement.

Selon un mode de réalisation préféré selon l'invention, le procédé selon l'invention comporte en outre le passage de la majeure partie de l'effluent de la zone de réaction dans une zone de décantation, ce qui permet l'obtention d'un effluent comprenant en majeure partie de l'acide liquide et d'une phase hydrocarbonée comprenant principalement de l'isoparaffine et de l'alkylat. Alors, selon une première option, ledit procédé est de préférence tel que le catalyseur alimentant la zone d'émulsion comprend la majeure partie de l'effluent comprenant en majeure partie de l'acide liquide qui sort de ladite zone de décantation. Selon une seconde option, indépendante ou non de la précédente, l'effluent comprenant en majeure partie de l'isoparaffine entrant en zone de premier mélange comprend en majeure partie une partie de ladite phase hydrocarbonée.

Selon le mode de réalisation préféré décrit ci-dessus, et indépendamment ou non de chacune des options décrites précédemment, ledit procédé peut de préférence comprendre en outre une zone de séparation qui permet l'obtention d'un effluent hydrocarboné contenant en majeure partie de l'isoparaffine et l'obtention d'un alkylat, produit de la réaction, ladite zone de séparation étant alimentée par une partie de la phase hydrocarbonée obtenue en sortie de la zone de décantation. Alors il est possible que le procédé selon l'invention soit tel que l'autre partie de ladite phase hydrocarbonée obtenue en sortie de la zone de décantation est comprise dans l'effluent comprenant en majeure partie de l'isoparaffine entrant en zone de premier mélange. Une autre possibilité, indépendante ou non de la possibilité précédente, est que le procédé selon l'invention soit tel que l'effluent hydrocarboné comprenant en majeure partie de l'isoparaffine qui alimente la zone d'émulsion comprend en majeure partie l'effluent hydrocarboné contenant en majeure partie de l'isoparaffine qui sort de la zone de séparation.

De plus, le procédé selon l'invention comprend un apport d'isoparaffine. Cet apport d'isoparaffine constitue l'alimentation du procédé selon l'invention en isoparaffine, qui se fait en stoechiométrie de la réaction d'alkylation par rapport à l'oléfine. De préférence, le procédé selon l'invention est tel que l'apport d'isoparaffine se fait par mélange de l'isoparaffine que l'on apporte dans la charge comprenant l'oléfine à convertir avant l'entrée de ladite charge en zone de premier mélange. Ainsi, de façon plus préférée, la charge qui entre en zone de premier mélange comprend principalement l'alimentation du procédé en oléfine(s) et en isoparaffine(s).

Le procédé selon l'invention comprend généralement un apport de catalyseur frais, c'est-à-dire de catalyseur n'ayant pas encore été soumis à quelque réaction chimique que ce soit ou de catalyseur ayant été soumis à une réaction chimique quelconque et ayant été régénéré dans une zone de régénération afin de restaurer ses propriétés catalytiques initiales, et un soutirage de catalyseur usé. De préférence, l'apport de catalyseur frais comprend en majeure partie du catalyseur issu d'une zone de régénération.

Le procédé selon l'invention est tel que ledit catalyseur acide liquide est généralement choisi dans le groupe formé par l'acide sulfurique et l'acide fluorhydrique, et de préférence ledit catalyseur acide liquide est l'acide sulfurique, qui de manière préférée est de titre supérieur à 96 % poids.

L'acide sulfurique liquide utilisé selon l'invention dans la zone de réaction à une température généralement inférieure à 0° C présente l'avantage sur l'acide sulfurique de titre 96 à 99% poids, actuellement utilisé dans les unités d'alkylation (dans les procédés conventionnels et opérant en zone de réaction à une température généralement supérieure à 0°C), de posséder une acidité supérieure ou égale tout en présentant un caractère oxydant beaucoup plus faible, et ainsi d'obtenir un alkylat dont l'indice d'octane est supérieur à ce qui est couramment obtenu avec lesdits procédés conventionnels. L'utilisation dudit catalyseur selon l'invention conduit donc à une réduction de la consommation de catalyseur et donc à une diminution des coûts des unités d'alkylation.

Mais le procédé de la présente invention s'applique à tout catalyseur acide liquide, comme par exemple un catalyseur comprenant au moins un acide choisi dans le groupe formé par l'acide sulfurique et l'acide fluorhydrique, auquel on a ajouté au moins un additif comme par exemple le composé HB(HSO₄)₄. Par exemple, le catalyseur utilisé selon le procédé de la présente invention comprend (en % poids) entre 0,4 et 68,8%, de préférence entre 0,4 et 60%, du composé HB(HSO₄)₄ et entre 31,2 et 99,6%, de préférence entre 40 et 99,6 %, du composé H₂SO₄, ledit catalyseur étant tel qu'il ne contient pas d'anhydride sulfurique (SO₃) non associé, c'est-à-dire n'ayant pas réagi avec l'acide borique mais pouvant éventuellement contenir de l'acide borique, en excès, non associé, c'est-à-dire n'ayant pas réagi avec l'anhydride sulfurique.

Le procédé selon la présente invention, plus particulièrement dans son mode de réalisation préféré qui comprend le passage de la majeure partie de l'effluent de la zone de réaction dans une zone de décantation, présente de nombreux avantages par rapport aux procédés d'alkylation connus de l'art antérieur, parmi lesquels on peut citer :
- l'utilisation d'une température de réaction basse, en particulier inférieure à 0°C,
- l'obtention de vitesses de décantation nettement plus grandes que celles obtenues quand la phase acide est la phase continue de l'émulsion,
- l'utilisation d'une température de mélange de l'oléfine et du catalyseur acide liquide très basse, et notamment plus basse que la température de la zone de réaction, en particulier inférieure à 0°C,
- la réalisation de l'opération de décantation à une température basse, avantage qui s'ajoute à celui de pouvoir réaliser un court temps de séjour dans ladite zone de décantation, ce qui permet de limiter considérablement l'importance des réactions secondaires se déroulant dans les décanteurs des procédés conventionnels.

Ainsi un mode de réalisation préféré selon l'invention concerne un procédé d'alkylation dans lequel on traite une charge comprenant d'une part au moins une isoparaffine choisie dans le groupe formé par l'isobutane et l'isopentane, de préférence l'isobutane et d'autre part au moins une oléfine contenant de 2 à 6, de préférence de 3 à 6 atomes de carbone par molécule, en présence d'un catalyseur acide liquide, ledit procédé étant tel qu'il comprend les étapes suivantes :
(1) la réalisation dans une zone (E) de l'émulsion de la majeure partie de l'acide liquide provenant d'une zone de décantation (D) décrit à l'étape (5) et de la majeure partie de l'effluent hydrocarboné comprenant en majeure partie de l'isoparaffine provenant de la zone de séparation isoparaffine-alkylat (S) décrit à l'étape (6), ladite majeure partie dudit effluent constituant la phase continue de ladite émulsion. L'effluent de la zone (E) est une émulsion d'acide dans un effluent liquide hydrocarboné comprenant en majeure partie de l'isoparaffine. Cette émulsion est constituée de fines gouttelettes d'acide dispersées dans une phase continue hydrocarbonée.
(2) le mélange dans une zone de premier mélange (M1) de la charge et d'une partie de la phase hydrocarbonée comprenant principalement l'isoparaffine et l'alkylat qui sort de la zone de décantation (D) décrite à l'étape (5), permettant l'obtention d'une charge diluée.

Les étapes (1) et (2) sont réalisées au moins en partie simultanément ou successivement, auquel cas l'ordre de réalisation desdites étapes intervient peu.
(3) le mélange dans une zone de second mélange (M2) de ladite charge diluée décrite à l'étape (2) et de ladite émulsion décrite à l'étape (1), permettant l'obtention d'un mélange d'acide dans un effluent liquide hydrocarboné qui est la phase continue de l'émulsion.
(4) la réalisation dans une zone (R) de la majeure partie de la réaction d'alkylation, la zone (R) étant alimentée par la majeure partie du mélange issu de la zone (M2) décrit à l'étape (3), de préférence introduite au moins à l'entrée de la zone (R). Le débit de l'effluent entrant dans la zone de réaction (R) est choisi de façon à ce que l'émulsion présente dans toute ladite zone reste constituée par des gouttelettes d'acide dispersée dans une phase continue hydrocarbonée.
(5) la séparation dans une zone de décantation (D) de la majeure partie de l'effluent sortant de la zone de réaction (R) décrite à l'étape (4). Ledit effluent est constitué d'une émulsion d'acide dans une phase continue hydrocarbonée. Dans la zone de décantation (D) est réalisée la séparation des deux phases acide et hydrocarbonée. La zone de décantation (D) comprend généralement au moins un décanteur. Le type de décanteur utilisé est choisi de façon à ce que le temps nécessaire pour réaliser l'opération de de décantation soit le plus faible possible, et que la température de la phase acide dans ledit décanteur reste inférieure à 0°C. La zone de décantation (D) permet l'obtention d'un effluent comprenant en majeure partie de l'acide liquide, la majeure partie dudit effluent étant recyclée vers la zone d'émulsion (E) décrite à l'étape (1), et l'obtention d'une phase hydrocarbonée comprenant principalement l'isoparaffine et l'alkylat, dont une partie est recyclée vers la zone de premier mélange (M1) décrite à l'étape (2).
(6) la séparation isoparaffine-alkylat dans une zone (S) de l'autre partie de la phase liquide hydrocarbonée comprenant principalement l'isoparaffine et l'alkylat issue de la zone de décantation (D) décrite à l'étape (5), en vue d'obtenir un effluent hydrocarboné comprenant en majeure partie de l'isoparaffine et dont la majeure partie est recyclée en zone (E) décrite à l'étape (1), éventuellement et de façon préférée un effluent hydrocarboné comprenant en majeure partie de la normale paraffine, obtenu à titre de purge, et un effluent hydrocarboné comprenant en majeure partie de l'alkylat, à titre de produit.

Dans le procédé d'alkylation d'isoparaffine(s) par au moins une oléfine selon la présente invention, les conditions opératoires, et plus particulièrement la température et la pression, sont choisies de façon à ce que le mélange réactionnel soit liquide.

Dans la mise en oeuvre du procédé selon l'invention, la réalisation des mélanges en zone (M1) et en zone (M2) peut se faire selon diverses techniques connues de l'homme du métier. Par exemple il est possible d'utiliser au moins un mélangeur statique en ligne (contenant des garnissages Sulzer par exemple). Dans tous les cas de figure, il est particulièrement avantageux d'utiliser des mélangeurs statiques en amont de la zone de réaction (R) de façon à assurer le meilleur contact possible entre les différents effluents avant leur introduction dans la zone de réaction (R). Le débit de l'effluent entrant dans la zone de réaction (R) est choisi de façon à ce que l'émulsion présente dans toute la zone de réaction (R) soit constituée par des gouttelettes d'acide dispersées dans une phase continue hydrocarbonée.

Un ou plusieurs réacteur(s) peu(ven)t être utilisé(s) pour la mise en oeuvre du procédé selon l'invention.

Dans une mise en oeuvre préférée selon l'invention, on soutire en continu ou en discontinu l'effluent comprenant en majeure partie du catalyseur du bas de la zone de décantation (D) et on introduit en continu ou en discontinu du catalyseur frais, à l'entrée de la zone d'émulsion (E) de façon à maintenir à un niveau constant la qualité de l'alkylat produit.

La température dans la zone d'émulsion (E) est généralement comprise entre -20 et +10°C, de préférence entre -10 et +10°C, et la pression est telle que tout hydrocarbure présent dans ladite zone est liquide.

La température dans la zone de second mélange (M2) est généralement comprise entre -20 et +10°C, de préférence entre -15 et 0°C, de façon encore plus préférée entre -10 et -3°C, et la pression est telle que tout hydrocarbure présent dans ladite zone est liquide.

La température dans la zone d'alkylation ou zone réactionnelle (R) est généralement comprise entre -20 et +10°C, de préférence entre -10 et +5°C, et de manière encore plus préférée comprise entre -10 et 0°C, et la pression est telle que tout hydrocarbure présent dans ladite zone est liquide.

La température dans la zone de décantation (D) est généralement comprise entre -20 et +10°C, de préférence entre -10 et +5°C, et de manière encore plus préférée comprise entre -10 et 0°C, et la pression est telle que tout hydrocarbure présent dans ladite zone est liquide.

Le temps de séjour des composés dans la zone de second mélange (M2) est très court, généralement entre 1 seconde et 2 minutes, de préférence entre 1 seconde et 1 minute, de façon à ce que la réaction d'alkylation ne puisse pas se produire de façon trop importante en zone (M2). Néanmoins, on considère que seulement la majeure partie de la réaction se produit en zone (R).

Le temps de séjour de l'effluent de la zone réactionnelle (R) dans la zone de décantation (D) est très court, plus court que pour les procédés d'alkylation conventionnels pour lesquels la phase continue de l'émulsion isoparaffine-acide est l'acide. Il est généralement compris entre 1 minute et 1 heure, de préférence entre 1 minute et 30 minutes, et d'une manière souvent préférée entre 1 et 5 minutes.

Le temps de séjour du catalyseur dans la zone d'émulsion (E) est généralement compris entre 1 seconde et 20 minutes, de préférence entre 1 seconde et 10 minutes.

Le temps de séjour des composés, et plus particulièrement du catalyseur, dans la zone de réaction (R) est généralement compris entre 1 minute et 1 heure, de préférence entre 1 minute et 30 minutes.

Le rapport des débits volumiques de l'effluent comprenant en majeure partie de l'acide provenant de la partie inférieure de la zone de décantation (D) et de l'effluent hydrocarboné comprenant en majeure partie de l'isoparaffine provenant de la tête de la zone de séparation (S), à l'entrée de la zone d'émulsion (E), est choisi de façon à ce que la proportion volumique d'acide dans ladite zone d'émulsion soit comprise entre 5 et 49%, de préférence entre 10 et 45% et d'une manière plus préférée entre 30 et 45%.

La proportion volumique d'acide au sein de la zone de réaction (R) est comprise entre 20 et 49%, de préférence entre 30 et 45%.

De préférence, la charge a été séchée sur tamis moléculaire et hydrogénée sélectivement avant son introduction dans la zone de premier mélange (M1), de manière à éliminer les composés très fortement insaturés susceptibles d'inhiber la phase catalytique.

De façon générale, la vitesse spatiale horaire, exprimée en volumes d'oléfine(s) introduite(s) par unité de volume du catalyseur présent dans la zone (R) et par heure, est comprise entre 0,1 et 5 h⁻¹, de préférence entre 0,2 et 2 h⁻¹.

De façon à limiter les réactions secondaires de dégradation des isoparaffines C₅-C₁₂ présentes dans l'effluent liquide passant au travers de la zone de réaction (R), on réalise de préférence l'opération de séparation (zone (S)) de façon à ce que le rapport des débits massiques de l'effluent hydrocarboné comprenant en majeure partie de l'isoparaffine (par exemple l'isobutane) qui sort en tête de la zone de séparation (S) et de l'effluent hydrocarboné comprenant en majeure partie de l'alkylat qui sort en fond de la zone (S) soit compris entre 5:1 et 100:1, et de préférence compris entre 10:1 et 30:1.

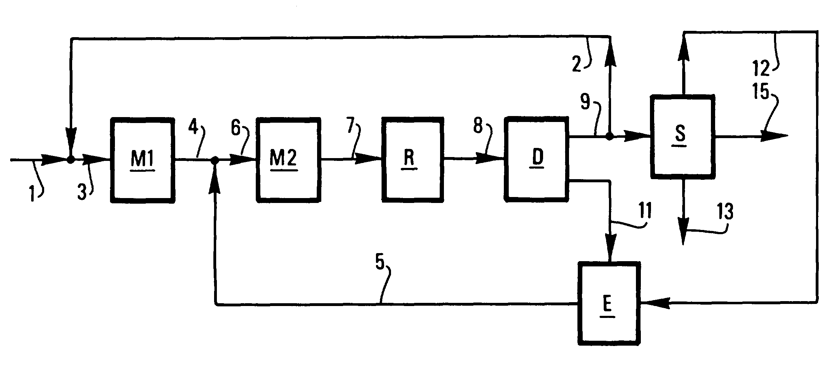

La figure jointe illustre l'invention, et plus précisément une des mises en oeuvre préférées du procédé selon l'invention, sans en limiter la portée.

La charge à convertir constituée par le mélange en phase liquide de la ligne (1), comprenant au moins une isoparaffine et au moins une oléfine contenant de 2 à 6 atomes de carbone par molécule, est mélangée avec l'effluent de la ligne (2), mélange d'isoparaffine et d'alkylat provenant d'une partie de l'effluent (9) sortant du décanteur (D) qui n'est pas envoyée vers la zone de séparation (S). Les lignes (1) et (2) mélangées forment ainsi la ligne (3) qui alimente le mélangeur statique (M1). L'effluent sortant de (M1) est injecté, après mélange avec l'effluent de la ligne (5) dans un second mélangeur statique (M2). L'effluent de la ligne (5) est constitué d'une émulsion d'acide dans l'isoparaffine, l'isoparaffine provenant de la tête de la zone de séparation (S) par la ligne (12) et l'acide provenant du fond de la zone de décantation (D) par la ligne (11). L'effluent sortant du mélangeur (M2), constitué d'une émulsion en phase hydrocarbonée continue, est introduit par la ligne (7) dans la zone de réaction (R). L'effluent sortant de la zone réactionnelle (R), c'est-à-dire l'émulsion d'acide dans une phase continue hydrocarbonée constituée majoritairement d'isoparaffine et d'alkylat, est introduit dans une zone de décantation (D). De la partie inférieure de la zone (D) est soutirée, par la ligne (11), l'acide, qui après mélange avec l'effluent (isoparaffine) de la ligne (12), est introduit à l'entrée d'une zone (E). De la partie supérieure de la zone de décantation (D) on soutire par la ligne (9) une phase hydrocarbonée contenant l'isoparaffine en excès et l'alkylat. Une partie de cet effluent est recyclée par la ligne (2) à l'entrée du premier mélangeur statique (M1 ) et l'autre partie est introduite dans une zone de séparation (S) isoparaffine-normale paraffine-alkylat. L'alkylat séparé dans la zone (S) est extrait de l'unité à titre de produit par la ligne (13). La normale paraffine est extraite latéralement de la zone (S) par la ligne (15) à titre de purge. La fraction liquide riche en isoparaffine extraite en tête de la zone (S) est recyclée vers l'entrée de la zone d'émulsion (E) par la ligne (12).

## Revendications

1. Procédé d'alkylation d'au moins une isoparaffine choisie dans le groupe formé par l'isobutane et l'isopentane par au moins une oléfine comprenant de 2 à 6 atomes de carbone par molécule en présence d'un catalyseur acide liquide, ledit procédé comprenant le mélange dans une zone de premier mélange de la charge comprenant l'oléfine à convertir et d'un effluent comprenant en majeure partie de l'isoparaffine, permettant l'obtention d'une charge diluée comprenant l'oléfine, ainsi que la formation dans une zone d'émulsion d'une émulsion dudit catalyseur dans un effluent hydrocarboné comprenant en majeure partie de l'isoparaffine, ledit effluent constituant la phase continue de l'émulsion ainsi formée, puis le mélange dans une zone de second mélange de la majeure partie de l'émulsion de l'acide dans l'effluent hydrocarboné et de la majeure partie de la charge diluée comprenant l'oléfine, suivie de la mise en oeuvre de la majeure partie de la réaction dans une zone de réaction qui est alimentée en la majeure partie dudit mélange, la température de la zone de second mélange étant inférieure à la température de la zone de réaction.

2. Procédé selon la revendication 1 comportant en outre le passage de la majeure partie de l'effluent de la zone de réaction dans une zone de décantation, ce qui permet l'obtention d'un effluent comprenant en majeure partie de l'acide liquide et d'une phase hydrocarbonée comprenant principalement de l'isoparaffine et de l'alkylat.

3. Procédé selon la revendication 2 tel que le catalyseur alimentant la zone d'émulsion comprend la majeure partie de l'effluent comprenant en majeure partie de l'acide liquide qui sort de ladite zone de décantation.

4. Procédé selon l'une des revendications 2 à 3 tel que l'effluent comprenant en majeure partie de l'isoparaffine entrant en zone de premier mélange comprend en majeure partie une partie de ladite phase hydrocarbonée.

5. Procédé selon l'une des revendications 2 à 4 comportant en outre une zone de séparation qui permet l'obtention d'un effluent hydrocarboné contenant en majeure partie de l'isoparaffine et l'obtention d'un alkylat, produit de la réaction, ladite zone de séparation étant alimentée par une partie de la phase hydrocarbonée obtenue en sortie de la zone de décantation.

6. Procédé selon la revendication 5 tel que l'effluent hydrocarboné comprenant en majeure partie de l'isoparaffine qui alimente la zone d'émulsion comprend en majeure partie l'effluent hydrocarboné contenant en majeure partie de l'isoparaffine qui sort de la zone de séparation.

7. Procédé selon les revendications 1 à 6 comprenant un apport d'isoparaffine.

8. Procédé selon l'une des revendications 1 à 7 comprenant un apport de catalyseur frais et un soutirage de catalyseur usé.

9. Procédé selon l'une des revendications 1 à 8 tel que ledit catalyseur acide liquide est choisi dans le groupe formé par l'acide sulfurique et l'acide fluorhydrique.

10. Procédé selon l'une des revendications 1 à 9 tel que ledit catalyseur acide liquide est l'acide sulfurique.

## Claims

1. A process for the alkylation of at least one isoparaffin selected from the group formed by isobutane and isopentane with at least one olefin containing 2 to 6 carbon atoms per molecule in the presence of a liquid acid catalyst, said process comprising mixing a feed comprising the olefin to be convened with an effluent comprising a major portion of isoparaffin in a first mixing zone, and forming an emulsion of said catalyst in a hydrocarbon effluent comprising a major portion of isoparaffin in an emulsifying zone, said effluent constituting the continuous phase of the emulsion thus formed, then mixing a major portion of the emulsion of acid in hydrocarbon effluent with a major portion of the diluted feed comprising the olefin in a second mixing zone, followed by carrying out the major portion of the reaction in a reaction zone which is supplied by the major portion of said mixture, the temperature of the second mixing zone being lower than the temperature of the reaction zone.

2. A process according to claim 1, further comprising passing a major portion of the effluent from the reaction zone to a settling zone to obtain an effluent comprising a major portion of the liquid acid and a hydrocarbon phase mainly comprising the isoparaffin and the alkylate.

3. A process according to claim 2, in which a major portion of the catalyst supplied to the emulsifying zone consists of the effluent comprising a major portion of the liquid acid leaving said settling zone.

4. A process according to claim 2 or claim 3, in which a major portion of the effluent comprising a major portion of isoparaffin entering the first mixing zone is constituted by a portion of said hydrocarbon phase.

5. A process according to any one of claims 2 to 4, further comprising a separation zone which produces a hydrocarbon effluent comprising a major portion of isoparaffin, and which produces an alkylate as a reaction product, said separation zone being supplied with a portion of the hydrocarbon phase obtained from the outlet to the settling zone.

6. A process according to claim 5, in which a major portion of the hydrocarbon effluent comprising a major portion of isoparaffin which supplies the emulsifying zone consists of the hydrocarbon effluent comprising a major portion of isoparaffin leaving the separation zone.

7. A process according to claims 1 to 6, comprising addition of isoparaffin.

8. A process according to any one of claims 1 to 7, comprising addition of fresh catalyst and extraction of used catalyst.

9. A process according to any one of claims 1 to 8, in which said liquid acid catalyst is selected from the group formed by sulphuric acid and hydrofluoric acid.

10. A process according to any one of claims 1 to 9, in which said liquid acid catalyst is sulphuric acid.

## Patentansprüche

1. Verfahren zur Alkylierung von wenigstens einem Isoparaffin, ausgewählt aus der Gruppe, bestehend aus Isobutan und Isopentan, durch wenigstens ein Olefin mit 2 bis 6 Kohlenstoffatomen im Molekül in Gegenwart eines flüssigen sauren Katalysators, wobei das Verfahren das Mischen in einer Zone des ersten Mischens der Beschickung umfaßt, die das umzuwandelnde Olefin und ein Effluens umfaßt, das zum größeren Teil das Isoparaffin umfaßt, was zuläßt, daß eine verdünnte Beschickung mit dem Olefin erhalten wird, wie auch das in einer Emulgierzone Erzeugen einer Emulsion des besagten Katalysators in einem Kohlenwasserstoffeffluens, das zum größeren Teil das Isoparaffin umfaßt, wobei das Effluens die kontinuierliche Phase der so gebildeten Emulsion ausmacht, des Weiteren das Mischen in einer Zone eines zweiten Mischens des größeren Teils der sauren Emulsion in dem Kohlenwasserstoffeffluens und des größeren Teils der verdünnten Beschickung, die das Olefin umfaßt, gefolgt vom Zuführen des größeren Teils der Reaktion in eine Reaktionszone, die zum größeren Teil mit der genannten Mischung beschickt ist, wobei die Temperatur der Zone des zweiten Mischens unterhalb der Temperatur der Reaktionszone liegt.

2. Verfahren nach Anspruch 1, des Weiteren umfassend das Weiterführen des größeren Teils des Effluens der Reaktionszone in eine Dekantierungszone, was zuläßt, daß man ein Effluens erhält, das zum größeren Teil die flüssige Säure und eine Kohlenwasserstoffphase umfaßt, die hauptsächlich das Isoparaffin und das Alkylat umfaßt.

3. Verfahren nach Anspruch 2, wobei der die Emulgierzone beschickende Katalysator den größeren Teil des Effluens umfaßt, das zum größeren Teil die flüssige Säure enthält, die die genannte Dekantierzone verläßt.

4. Verfahren nach einem der Ansprüche 2 bis 3, wobei das Effluens, das zum größeren Teil das Isoparaffin umfaßt, das die Zone des ersten Mischens betritt, zum größeren Teil einen Teil der genannten Kohlenwasserstoffphase umfaßt.

5. Verfahren nach einem der Ansprüche 2 bis 4, des Weiteren umfassend eine Trennzone, die zuläßt, daß man ein Kohlenwasserstoffeffluens erhält, das zum größeren Teil das Isoparaffin enthält, und daß man ein Akylat erhält, das Produkt der Reaktion, wobei die genannte Trennzone mit einem Teil der Kohlenwasserstoffphase beschickt wird, die am Ausgang der Dekantierzone erhalten wird.

6. Verfahren nach Anspruch 5, wobei das Kohlenwasserstoffeffluens, das zum größeren Teil das Isoparaffin umfaßt, das die Emulgierzone beschickt, zum größeren Teil das Kohlenwasserstoffeffluens umfaßt, das zum größeren Teil das Isoparaffin umfaßt, das die Trennzone verläßt.

7. Verfahren gemäß den Ansprüchen 1 bis 6, umfassend die Zufuhr eines Isoparaffins.

8. Verfahren nach einem der Ansprüche 1 bis 7, umfassend die Zufuhr frischen Katalysators und das Abziehen des verbrauchten Katalysators.

9. Verfahren nach einem der Ansprüche 1 bis 8, wobei der flüssige saure Katalysator ausgewählt ist aus der Gruppe, bestehend aus Schwefelsäure und Fluorwasserstoffsäure.

10. Verfahren nach einem der Ansprüche 1 bis 9, wobei der flüssige saure Katalysator Schwefelsäure ist.
